# EUROPÄISCHE PATENTANMELDUNG

(11) **EP 2 468 195 A2**
(43) Veröffentlichungstag der Anmeldung: **27.06.2012**
(21) Anmeldenummer: 11010029.4
(22) Anmeldetag: 21.12.2011
(51) Int. Cl.: A61B 17/062

(54) **Medizinischer Nadelhalter**

(30) Priorität: 23.12.2010 DE 102010055806
(71) Anmelder: Karl Storz GmbH & Co. KG, 78532 Tuttlingen (DE)
(72) Erfinder: Haseke, Nicolas, Dr., 80797 München (DE); Schneider, Sven, 78532 Tuttlingen (DE); Bacher, Uwe, 78532 Tuttlingen (DE)
(74) Vertreter: Hofmeister, Frank

(57) **Zusammenfassung**

Die Erfindung betrifft einen medizinischen Nadelhalter mit einem Schaft (2), mit einem am distalen Ende des Schaftes (2) angeordneten, aus zwei Maulteilen (6a, 6b) bestehenden Werkzeug (6), wobei ein Maulteil (6b) des Werkzeugs (6) gegenüber dem anderen Maulteil (6a) des Werkzeugs (6) verschwenkbar ausgebildet ist, sowie mit einer am proximalen Ende des Schaftes (2) angeordneten, mindestens zwei Griffteile (3, 4) aufweisenden Handhabe (5), wobei das verschwenkbare Maulteil (6b) des Werkzeugs (6) über ein verstellbar ausgebildetes Griffteil (3) der Handhabe (5) betätigbar ist, wobei mindestens ein Teilbereich (6c) mindestens eines Maulteils (6a oder 6b) des Werkzeugs (6) in Längsrichtung des Schaftes (2) relativ zu dem anderen Maulteil (6b oder 6a) des Werkzeugs (6) verschiebbar ausgebildet ist. Um einen medizinischen Nadelhalter (1) zu schaffen, der eine einfache Positionierung der Nadel (9) im Werkzeug (6) ermöglicht, wird erfindungsgemäß vorgeschlagen, dass das verschiebbare Maulteil (6a) oder der verschiebbare Teilbereich (6c) des Maulteils (6a) über ein Federelement in eine Ausgangsposition vorgespannt ist.

## Beschreibung

Die Erfindung betrifft einen medizinischen Nadelhalter mit einem Schaft, mit einem am distalen Ende des Schaftes angeordneten, aus zwei Maulteilen bestehenden Werkzeug, wobei ein Maulteil des Werkzeugs gegenüber dem anderen Maulteil des Werkzeugs verschwenkbar ausgebildet ist, sowie mit einer am proximalen Ende des Schaftes angeordneten, mindestens zwei Griffteile aufweisenden Handhabe, wobei das verschwenkbare Maulteil des Werkzeugs über ein verstellbar ausgebildetes Griffteil der Handhabe betätigbar ist, wobei mindestens ein Teilbereich mindestens eines Maulteils des Werkzeugs in Längsrichtung des Schaftes relativ zu dem anderen Maulteil des Werkzeugs verschiebbar ausgebildet ist.

Medizinische Nadelhalter sind in verschiedenen Ausführungsformen bekannt. Die in der Regel sichelförmig gebogenen chirurgischen Nadeln werden von dem Werkzeug ergriffen und klemmend zwischen den Maulteilen des Werkzeugs gehalten. Um die solchermaßen gehaltene Nadel in die richtige Lage zum Vernähen einer Operationsstelle auszurichten, muss der Operateur die Nadel entweder manuell ausrichten oder aber mittels einer weiteren Fasszange in die erforderliche Lage zwischen den Maulteilen des Werkzeugs überführen.

Ein gattungsgemäßer medizinischer Nadelhalter ist beispielsweise aus der DE 91 09 097 U1 bekannt.

Davon ausgehend liegt der Erfindung die **Aufgabe** zugrunde, einen medizinischen Nadelhalter zu schaffen, der eine einfache Positionierung der Nadel im Werkzeug ermöglicht.

Die **Lösung** dieser Aufgabenstellung ist erfindungsgemäß dadurch gekennzeichnet, dass das verschiebbare Maulteil oder der verschiebbare Teilbereich des Maulteils über ein Federelement in eine Ausgangsposition vorgespannt ist.

Durch die Ausgestaltung der Maulteile des Werkzeugs, wonach zumindest ein Teilbereich mindestens eines Maulteils des Werkzeugs in Längsrichtung des Schaftes relativ zu dem anderen Maulteil des Werkzeugs verschiebbar ausgebildet ist, ist es erstmalig möglich, die Lage der zwischen den Maulteilen gehaltenen chirurgischen Nadel ohne Zuhilfenahme eines zusätzlichen Werkzeugs oder manuelles Ergreifen auszurichten. Um nach dem Loslassen der chirurgischen Nadel dem verschiebbaren Maulteil wieder den gleichen Bewegungsspielraum zum erneuten Ergreifen der Nadel zu ermöglichen, ist das verschiebbare Maulteil oder der verschiebbare Teilbereich des Maulteils erfindungsgemäß über ein Federelement in eine Ausgangsposition vorgespannt.

Gemäß einer praktischen Ausführungsform der Erfindung wird vorgeschlagen, dass mindestens ein Teilbereich des nicht verschwenkbaren Maulteils des Werkzeugs in Längsrichtung des Schaftes verschiebbar ausgebildet ist. Die Längsverschiebbarkeit des nicht verschwenkbaren Maulteils ist konstruktiv besonders einfach herzustellen, da dieses Maulteil im Gegensatz zum verschwenkbaren Maulteil keine weiteren Bewegungen ausführen muss.

Das Verschieben des Maulteils oder des Teilbereichs eines Maulteils in Längsrichtung des Schaftes erfolgt erfindungsgemäß über einen an der Handhabe angeordneten Antrieb. Mit einer bevorzugten Ausführungsform der Erfindung wird vorgeschlagen, dass der Antrieb als Stellrad ausgebildet ist.

Zur Ausbildung des axial verschiebbaren Maulteils wird mit der Erfindung weiterhin vorgeschlagen, dass das verschiebbare Maulteil oder der verschiebbare Teilbereich des Maulteils das distale Ende eines in Längsrichtung des Schaftes verschiebbaren und am Schaft führend gelagerten Schlittens bildet. Der geführte Schlitten gewährleistet eine exakte und verkippsichere Axialverschiebung des Maulteils.

Zum Antrieb des Schlittens in Längsrichtung des Schaftes wird die vom als Stellrad ausgebildeten Antrieb erzeugte Rotationsbewegung in eine reine axiale Längsbewegung des Schlittens überführt. Diese Umsetzung kann beispielsweise durch einen reinen Reibschluss oder aber auch durch eine Verzahnung von Stellrad und Schlitten bewirkt werden.

Um den Verschleiß der Greiffläche des verschiebbaren Maulteils im Kontaktbereich zwischen der chirurgischen Nadel und dem verschiebbaren Maulteil zu minimieren, besteht zumindest der zur Aufnahme einer zu haltenden chirurgischen Nadel dienende Teilbereich der Greiffläche des Schlittens aus einem Hartmetall.

Weiterhin wird mit der Erfindung vorgeschlagen, dass zum Antrieb des in Längsrichtung des Schaftes verschiebbaren Teilbereichs eines Maulteils am distalen Ende dieses Maulteils ein vorzugsweise als Stellschraube ausgebildeter Antrieb angeordnet ist.

Damit die chirurgische Nadel einerseits leicht drehbar zwischen den beiden Maulteilen des Werkzeugs lagerbar ist und andererseits ein einfaches und sicheres Fixieren der chirurgischen Nadel zwischen den Maulteilen des Werkzeugs möglich ist, wird gemäß einer ersten Ausführungsform der Erfindung vorgeschlagen, dass auf der zur Aufnahme einer zu haltenden chirurgischen Nadel dienenden Greiffläche mindestens eines Maulteils eine in Längsrichtung des Maulteils verlaufende auswechselbare Kunststoffschiene angeordnet ist. Die Schiene minimiert einerseits die Kontaktfläche der Nadel mit den Maulteilen und erlaubt andererseits aufgrund der Verformbarkeit des Kunststoffmaterials ein sicheres Fixieren der Nadel, wenn die beiden Maulteile über die Handhabe zusammengedrückt werden.

Mit einer zweiten erfindungsgemäßen Ausführungsform wird vorgeschlagen, dass auf die zur Aufnahme einer zu haltenden chirurgischen Nadel dienende Greiffläche mindestens eines Maulteils eine auswechselbare Kunststoffabdeckung aufsetzbar ist. Diese Variante ist besonders schnell und einfach montierbar und auswechselbar, da die Kunststoffabdeckung von außen auf das mindestens eine Maulteil aufgesetzt wird.

Weiterhin wird mit einer dritten alternativen Ausführungsform vorgeschlagen, dass zumindest die zur Aufnahme einer zu haltenden chirurgischen Nadel dienenden Greiffläche mindestens eines Maulteils aus einem auswechselbaren Kunststoffmaterial besteht. Bei dieser Ausführungsform besteht zumindest die Greiffläche, wenn nicht sogar das gesamte Maulteil aus einem Kunststoffmaterial.

Schließlich wird mit der Erfindung vorgeschlagen, dass die Griffteile der Handhabe in ihrer jeweiligen Position zueinander fixierbar sind, um einerseits einen stets sicheren Halt der chirurgischen Nadel im Werkzeug zu gewährleisten und andererseits den Bediener zu entlasten, so dass dieser die Griffteile nicht fortwährend betätigen muss.

Weitere Merkmale und Vorteile der Erfindung ergeben sich anhand der zugehörigen Zeichnungen, in denen verschiedene Ausführungsbeispiele eines erfindungsgemäßen medizinischen Nadelhalters nur beispielhaft dargestellt sind, ohne die Erfindung auf diese Ausführungsbeispiele zu beschränken. In den Zeichnungen zeigt:
- Fig. 1: eine Seitenansicht einer ersten Ausführungsform eines erfindungsgemäßen medizinischen Nadelhalters in einer ersten Arbeitsstellung der Maulteile;
- Fig. 2: eine Darstellung gemäß Fig. 1, jedoch die Maulteile in einer zweiten Arbeitsstellung darstellend;
- Fig. 3: eine perspektivische Ansicht des distalen Endes einer zweiten Ausführungsform eines erfindungsgemäßen medizinischen Nadelhalters in der geöffneten Stellung;
- Fig. 4: einen vergrößerten Längsschnitt entlang der Linie IV-IV gemäß Fig. 3;
- Fig. 5: eine vergrößerte Darstellung des Details V gemäß Fig. 1, eine erste Ausführungsform der Maulteile darstellend;
- Fig. 6: einen Schnitt entlang der Linie VI-VI gemäß Fig. 5;
- Fig. 7: eine vergrößerte Darstellung des Details VII gemäß Fig. 1, eine zweite Ausführungsform der Maulteile darstellend;
- Fig. 8: einen Schnitt entlang der Linie VIII-VIII gemäß Fig. 7 und
- Fig. 9: eine vergrößerte Darstellung des Details IX gemäß Fig. 1, eine dritte Ausführungsform der Maulteile darstellend.

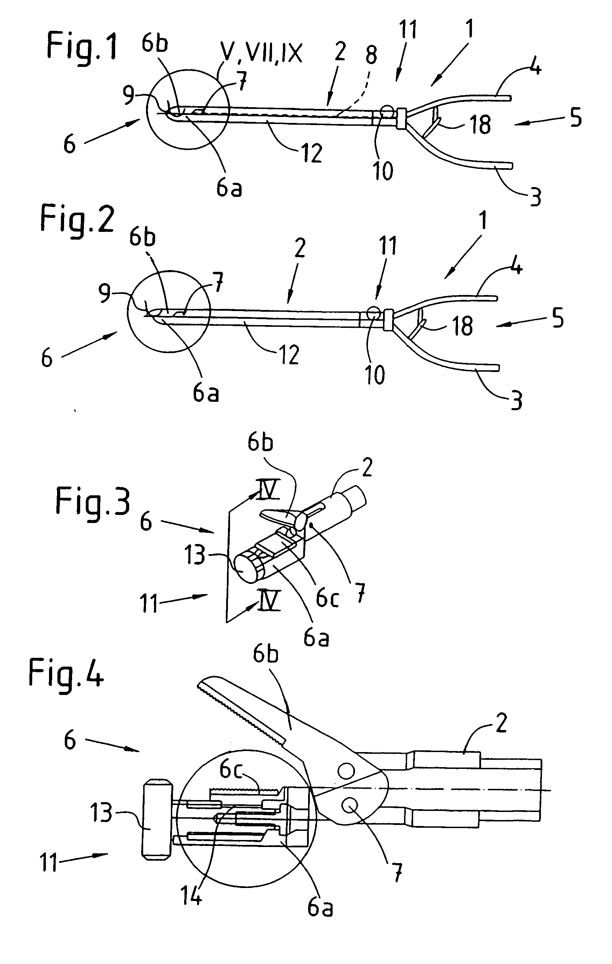

Die Abbildungen Fig. 1 und 2 zeigen die Seitenansichten eines medizinischen Nadelhalters 1.

Der dargestellte medizinische Nadelhalter 1 besteht im Wesentlichen aus einem hohlen Schaft 2, an dessen proximalem Ende eine zwei Griffteilen 3 und 4 aufweisende Handhabe 5 angeordnet ist, wobei bei der dargestellten Ausführungsform das Griffteil 3 gegenüber dem anderen Griffteil 4 verschwenkbar an der Handhabe 5 gelagert ist.

Am distalen Ende des Schaftes 2 ist ein Werkzeug 6 angeordnet, welches beim dargestellten Ausführungsbeispiel aus einem starr mit dem Schaft 2 verbundenen Maulteil 6a sowie einem um eine Schwenkachse 7 verschwenkbaren Maulteil 6b besteht. Zum Öffnen und Schließen der Maulteile 6a und 6b des Werkzeugs 6 über die Betätigung des verschwenkbaren Griffteils 3 der Handhabe 5 stehen das verschwenkbare Griffteile 3 und das verschwenkbare Maulteil 6b über eine im hohlen Schaft 2 gelagerte Zug-/Druckstange 8 miteinander in Wirkverbindung.

Wie insbesondere aus den Abbildungen Fig. 1 bis 3 ersichtlich, sind die Maulteile 6a und 6b des Werkzeugs 6 nicht nur so ausgebildet, dass das Maulteil 6b gegenüber dem Maulteil 6a verschwenkbar ist, sondern zusätzlich auch das Maulteil 6a oder ein Teilbereich 6c des Maulteils 6a in Längsrichtung des Schaftes 2 gegenüber dem Maulteil 6b verschiebbar ausgebildet ist.

Alternativ zu der dargestellten Ausbildung der Maulteile 6a und 6b des Werkzeugs 6 ist es selbstverständlich auch möglich, dass das obere Maulteil 6b starr, das heißt nicht verschwenkbar ausgebildet ist und das untere Maulteil 6a verschwenkbar ausgebildet ist. Ebenso können das Maulteil 6b oder ein Teilbereich des Maulteils 6b gegenüber dem Maulteil 6a in Längsrichtung des Schaftes 2 verschiebbar ausgebildet sein. Auch die Relativverschiebbarkeit beider Maulteile 6a und 6b zueinander ist eine mögliche Konstruktionsvariante.

Durch diese Ausgestaltung der Maulteile 6a und 6b des Werkzeugs 6, wonach zumindest ein Teilbereich 6c mindestens eines Maulteils 6a oder 6b des Werkzeugs 6 in Längsrichtung des Schaftes 2 relativ zu dem anderen Maulteil 6b oder 6a des Werkzeugs 6 verschiebbar ausgebildet ist, ist es möglich, die Lage einer zwischen den Maulteilen 6a und 6b gehaltenen chirurgischen Nadel 9 ohne Zuhilfenahme eines zusätzlichen Werkzeugs oder manuelles Ergreifen auszurichten, da das Verschieben der Maulteile 6a und 6b relativ zueinander ein Verdrehen der Nadel 9 um ihre Längsachse bewirkt.

Bei dem in den Abbildungen Fig. 1 und 2 dargestellten medizinischen Nadelhalter 1 erfolgt das Verschieben des starren, das heißt nicht verschwenkbaren Maulteils 6a in Längsrichtung des Schaftes 2 dadurch, dass der gesamte untere Teil des Schaftes 2, dessen distales Ende das Maulteil 6a bildet, über einen als Stellrad 10 ausgebildeten Antrieb 11 relativ zu dem oberen Teil des Schaftes 2 verschiebbar ausgebildet ist. Hierzu bildet der verschiebbare untere Teil des Schaftes 2 einen Schlitten 12, der führend am oberen Teil des Schaftes 2 gelagert ist. Der geführte Schlitten 12 gewährleistet eine exakte und verkippsichere Axialverschiebung des Maulteils 6a.

Zum Antrieb des Schlittens 12 in Längsrichtung des Schaftes 2 wird die vom als Stellrad 10 ausgebildeten Antrieb 11 erzeugte Rotationsbewegung in eine reine axiale Längsbewegung des Schlittens 12 überführt. Diese Umsetzung kann beispielsweise durch einen reinen Reibschluss oder aber auch durch eine Verzahnung von Stellrad 10 und Schlitten 12 bewirkt werden.

Bei der in Fig. 3 und 4 dargestellten alternativen Ausführungsform zur Ausbildung der Relativverschiebbarkeit der Maulteile 6a und 6b des Werkzeugs 6 ist nicht das gesamte Maulteil 6a in Längsrichtung des Schaftes 2 verschiebbar ausgebildet, sondern nur der zur klemmenden Aufnahme der Nadel 9 dienende Teilbereich 6c des Maulteils 6a.

Bei dieser Ausführungsform ist der Antrieb 11 des verschiebbaren Teilbereichs 6c des Maulteils 6a als Stellschraube 13 ausgebildet, die am distalen Ende des Maulteils 6a angeordnet ist und mit dem verschiebbaren Teilbereich 6c über eine Verzahnung 14 in Wirkverbindung steht.

Um den Verschleiß der Greiffläche des verschiebbaren Maulteils 6a oder des verschiebbaren Teilbereichs 6c im Kontaktbereich mit der chirurgischen Nadel 9 zu minimieren, besteht zumindest der zur Aufnahme einer zu haltenden chirurgischen Nadel 9 dienende Teilbereich der Greiffläche mindestens eines Maulteils 6a, 6b aus einem Hartmetall.

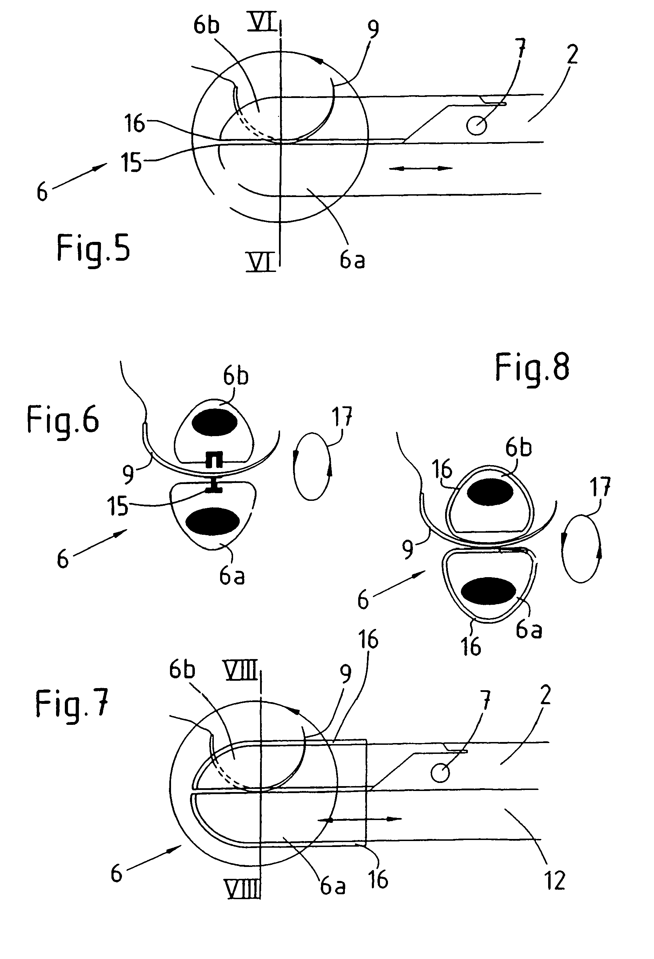

In den Abbildungen Fig. 5 bis 9 sind drei verschiedene Ausführungsformen zur Ausbildung der Maulteile 6a und 6b dargestellt, wie sie bei der Ausbildung des vollständig verschiebbaren Maulteils 6a gemäß Fig. 1 und 2 zur Anwendung kommen können.

Damit die chirurgische Nadel 9 einerseits leicht drehbar zwischen den beiden Maulteilen 6a und 6b des Werkzeugs 6 lagerbar ist und andererseits ein einfaches und sicheres Fixieren der chirurgischen Nadel 9 zwischen den Maulteilen 6a und 6b des Werkzeugs 6 möglich ist, ist bei diesen Ausführungsformen auf der zur Aufnahme einer zu haltenden chirurgischen Nadel 9 dienenden Greiffläche mindestens eines Maulteils 6a oder 6b ein Kunststoffmaterial angeordnet ist. Aufgrund der Verformbarkeit des Kunststoffmaterials wird ein sicheres Fixieren der Nadel 9 gewährleistet, wenn die beiden Maulteile 6a und 6b über die Handhabe 5 zusammengedrückt werden.

Bei der in Fig. 5 und 6 dargestellten ersten Ausführungsform sind auf beiden zur Aufnahme der zu haltenden chirurgischen Nadel 9 dienenden Greifflächen der Maulteile 6a und 6b in Längsrichtung der Maulteile 6a und 6b verlaufende auswechselbare Schienen 15 aus einem Kunststoffmaterial angeordnet. Diese Schienen 15 minimieren die Kontaktfläche der Nadel 9 mit den Maulteilen 6a und 6b und erleichtern so das Verdrehen der Nadel 9 beim Verschieben des Maulteils 6a.

Gemäß der in Fig. 7 und 8 dargestellten zweiten Ausführungsform sind auf die zur Aufnahme der zu haltenden chirurgischen Nadel 9 dienenden Greifflächen der Maulteile 6a und 6b auswechselbare Abdeckungen 16 aus einem Kunststoffmaterial aufsetzbar. Diese Variante ist besonders schnell und einfach montierbar und auswechselbar, da die Kunststoffabdeckungen 16 von außen auf die Maulteile 6a und 6b aufgesetzt werden.

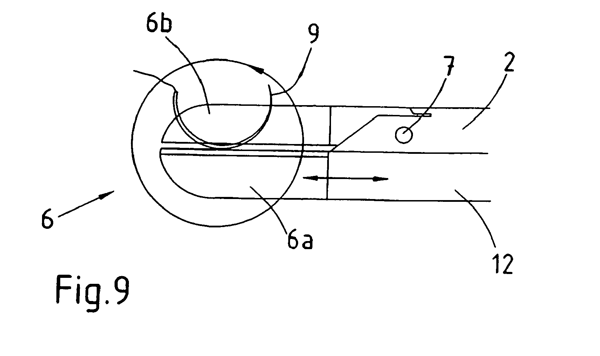

Bei der in Fig. 9 dargestellten dritten Ausführungsform bestehen die gesamten Maulteile 6a und 6b oder zumindest die zur Aufnahme der zu haltenden chirurgischen Nadel 9 dienenden Greifflächen der Maulteile 6a und 6b aus einem auswechselbaren Kunststoffmaterial.

Die Handhabung des zuvor beschriebenen und in den Abbildungen Fig. 1 bis 9 dargestellten medizinischen Nadelhalters 1 geschieht wie folgt:

Ausgehend von der in Fig. 3 dargestellten geöffneten Stellung des Werkzeugs 6 wird der Nadelhalter 1 vom Bediener so platziert, dass die chirurgische Nadel 9 zwischen den Maulteilen 6a und 6b des Werkzeugs 6 zu liegen kommt. Anschließend drückt der Bediener die Griffteile 3 und 4 der Handhabe 5 zusammen, so dass das verschwenkbare Maulteil 6b geschlossen und die Nadel zwischen den beiden Maulteilen 6a und 6b klemmend gehalten wird.

Die, wie aus Fig. 6 und 8 ersichtlich, in der Regel sichelförmig gebogene Nadel 9 muss zum Vernähen einer Operationsstelle so im Nadelhalter 1 ausgerichtet werden, dass der Operateur die Nadel 9 im richtigen Winkel in das zu vernähende Gewebe einstechen kann.

Das Ausrichten der zwischen den Maulteilen 6a und 6b gehaltenen Nadel 9 geschieht bei den beschriebenen Nadelhaltern 1 dadurch, dass entweder das gesamte Maulteil 6a oder nur ein Teilbereich 6c des Maulteils 6a gegenüber dem anderen Maulteil 6b in Längsrichtung des Schaftes 2 verschoben wird, was eine Rotation der Nadel 9 um ihre Längsachse bewirkt, wie dies in Fig. 6 und 8 durch den Pfeil 17 angedeutet ist.

Um einerseits einen stets sicheren Halt der chirurgischen Nadel 9 im Werkzeug 6 zu gewährleisten und andererseits den Bediener zu entlasten, so dass dieser die Griffteile 3 und 4 der Handhabe 5 nicht fortwährend betätigen muss, sind die Griffteile 3 und 4 der Handhabe 5 über einen Arretiermechanismus 18 in ihrer jeweiligen Position zueinander fixierbar sind, wie dies den Abbildungen Fig. 1 und 2 zu entnehmen ist.

Mittels dieses Arretiermechanismus 18 ist es möglich, die Nadel in einem ersten Schritt nur so zwischen den Maulteilen 6a und 6b festzulegen, dass diese zum Ausrichten noch verdrehbar ist und erst nachfolgend das endgültige Klemmen der Nadel 9 in der richtigen Lage erfolgt.

Um nach dem Loslassen der chirurgischen Nadel 9 dem verschiebbaren Maulteil 6a bzw. dem verschiebbaren Teilbereich 6c des Maulteils 6a wieder den gleichen Bewegungsspielraum zum erneuten Ergreifen der Nadel 9 zu ermöglichen, ist das verschiebbare Maulteil 6a bzw. der verschiebbare Teilbereich 6c des Maulteils 6a über ein nicht dargestelltes Federelement in eine Ausgangsposition vorgespannt.

Ein solchermaßen ausgebildeter medizinischer Nadelhalter 1 zeichnet sich dadurch aus, dass der eine einfache Positionierung der Nadel 9 im Werkzeug 6 ohne Zuhilfenahme eines weiteren Instruments ermöglicht.

### Bezugszeichenliste

- 1: medizinischer Nadelhalter
- 2: Schaft
- 3: Griffteil (verschwenkbar)
- 4: Griffteil (starr)
- 5: Handhabe
- 6: Werkzeug
- 6a: starres Maulteil
- 6b: verschwenkbares Maulteil
- 6c: Teilbereich (verschiebbar)
- 7: Schwenkachse
- 8: Zug-/Druckstange
- 9: Nadel
- 10: Stellrad
- 11: Antrieb
- 12: Schlitten
- 13: Stellschraube
- 14: Verzahnung

- 15: Schiene
- 16: Abdeckung
- 17: Pfeil (Rotation)
- 18: Arretiermechanismus

## Patentansprüche

1. Medizinischer Nadelhalter mit einem Schaft (2), mit einem am distalen Ende des Schaftes (2) angeordneten, aus zwei Maulteilen (6a, 6b) bestehenden Werkzeug (6), wobei ein Maulteil (6b) des Werkzeugs (6) gegenüber dem anderen Maulteil (6a) des Werkzeugs (6) verschwenkbar ausgebildet ist, sowie mit einer am proximalen Ende des Schaftes (2) angeordneten, mindestens zwei Griffteile (3, 4) aufweisenden Handhabe (5), wobei das verschwenkbare Maulteil (6b) des Werkzeugs (6) über ein verstellbar ausgebildetes Griffteil (3) der Handhabe (5) betätigbar ist, wobei mindestens ein Teilbereich (6c) mindestens eines Maulteils (6a oder 6b) des Werkzeugs (6) in Längsrichtung des Schaftes (2) relativ zu dem anderen Maulteil (6b oder 6a) des Werkzeugs (6) verschiebbar ausgebildet ist,
**dadurch gekennzeichnet,**
**dass** das verschiebbare Maulteil (6a) oder der verschiebbare Teilbereich (6c) des Maulteils (6a) über ein Federelement in eine Ausgangsposition vorgespannt ist.

2. Medizinisches Instrument nach Anspruch 1, **dadurch gekennzeichnet, dass** mindestens ein Teilbereich (6c) des nicht verschwenkbaren Maulteils (6a) des Werkzeugs (6) in Längsrichtung des Schaftes (2) verschiebbar ausgebildet ist.

3. Medizinisches Instrument nach Anspruch 1 oder 2, **dadurch gekennzeichnet, dass** das Verschieben in Längsrichtung des Schaftes (2) über einen an der Handhabe (5) angeordneten Antrieb (11) erfolgt.

4. Medizinisches Instrument nach Anspruch 3, **dadurch gekennzeichnet, dass** der Antrieb (11) als Stellrad (10) ausgebildet ist.

5. Medizinisches Instrument nach einem der Ansprüche 2 bis 4, **dadurch gekennzeichnet, dass** das verschiebbare Maulteil (6a) oder der verschiebbare Teilbereich (6c) des Maulteils (6a) das distale Ende eines in Längsrichtung des Schaftes (2) verschiebbaren und am Schaft (2) führend gelagerten Schlittens (12) bildet.

6. Medizinisches Instrument nach Anspruch 4 oder 5, **dadurch gekennzeichnet, dass** die Rotationsbewegung des Stellrads (10) in eine Längsbewegung des Schlittens (12) überführbar ist.

7. Medizinisches Instrument nach Anspruch 5 oder 6, **dadurch gekennzeichnet, dass** zumindest der zur Aufnahme einer zu haltenden chirurgischen Nadel (9) dienende Teilbereich der Greiffläche des Schlittens (12) aus einem Hartmetall besteht.

8. Medizinisches Instrument nach Anspruch 1 oder 2, **dadurch gekennzeichnet, dass** das Verschieben des Teilbereichs (6c) des Maulteils (6a) Längsrichtung des Schaftes (2) über einen am distalen Ende des Maulteils (6a) angeordneten Antrieb (11) erfolgt.

9. Medizinisches Instrument nach Anspruch 8, **dadurch gekennzeichnet, dass** der Antrieb (11) als Stellschraube (13) ausgebildet ist.

10. Medizinisches Instrument nach einem der Ansprüche 1 bis 9 **dadurch gekennzeichnet, dass** auf der zur Aufnahme einer zu haltenden chirurgischen Nadel (9) dienenden Greiffläche mindestens eines Maulteils (6a, 6b) eine in Längsrichtung des Maulteils (6a, 6b) verlaufende auswechselbare Schiene (15) aus einem Kunststoffmaterial angeordnet ist.

11. Medizinisches Instrument nach einem der Ansprüche 1 bis 9 **dadurch gekennzeichnet, dass** auf die zur Aufnahme einer zu haltenden chirurgischen Nadel (9) dienende Greiffläche mindestens eines Maulteils (6a, 6b) eine auswechselbare Abdeckung (16) aus einem Kunststoffmaterial aufsetzbar ist.

12. Medizinisches Instrument nach einem der Ansprüche 1 bis 9 **dadurch gekennzeichnet, dass** zumindest die zur Aufnahme einer zu haltenden chirurgischen Nadel (9) dienenden Greiffläche mindestens eines Maulteils (6a, 6b) aus einem auswechselbaren Kunststoffmaterial besteht.

13. Medizinisches Instrument nach einem der Ansprüche 1 bis 12, **dadurch gekennzeichnet, dass** die Griffteile (3, 4) der Handhabe (5) in ihrer jeweiligen Position zueinander fixierbar sind.
